# EUROPEAN PATENT APPLICATION

(11) **EP 3 219 343 A1**
(43) Date of publication of application: **20.09.2017**
(21) Application number: 17157036.9
(22) Date of filing: 21.02.2017
(51) Int. Cl.: A61M 5/00, A61M 1/00

(54) **ASSEMBLY FOR HANDLING A CONTAINER**

(30) Priority: 18.03.2016 BE 201605197
(71) Applicant: Nougimmo SPRL, 1420 Braine l'Alleud (BE)
(72) Inventor: MANCIONE, Antonio, 59264 ONNAING (FR)
(74) Representative: Gevers Patents

(57) **Abstract**

This invention relates to a suction cup (2), a negative pressure transfer means (3), a handling assembly (1) comprising the suction cup (2) and the negative pressure transfer means (3), a tray for securing the handling assemblies (1) and a container grasping system (10) comprising the tray and handling assemblies (1).

The suction cup (2) comprises channels that are secured by suction onto a perimeter (12) of an aperture of a hole (11) of the container (10) and a protrusion (28) positioned internally in relation to the channels that closes a hole (11) of the container (10). The suction cup (2) enables the container (10) to be grasped and handled while closing its hole (11). The use of numerous suction cups (2) in parallel enables numerous containers (10) to be handled in parallel.

## Description

### Technical field

According to a first aspect, this invention relates to a handling assembly capable of handling a container. According to a second aspect, this invention relates to a tray for bearing the handling assemblies. According to a third aspect, this invention relates to a container grasping system.

### Prior art

In the pharmaceutical industry, syringes arrive in a nest, which comprises several tens of syringes, and must be removed from said nest.

One solution for removing the syringes from the nest is disclosed in document EP 1 088 566 B1. The syringes can be removed from a box by sliding the teeth of a comb between the syringes so that the collars of the syringes rest on the teeth of the comb.

A first drawback of this known solution is that adjacent syringes supported by the same two teeth hit against each other, which creates microscopic cracks in the syringes. A second drawback is that the syringe holes remain open while the syringes are handled, thus resulting in a risk of the liquid present in the holes escaping or evaporating. Moreover, the comb must remain in a perfectly horizontal position to prevent the liquid from escaping, potentially to other syringes. A third drawback is that, if the comb is inadvertently inclined with its teeth pointing downwards, the syringes are released and break, spreading their contents and creating numerous glass shards.

### Summary of the invention

According to a first aspect, one of the purposes of the invention is to provide an assembly for handling containers, that prevents the containers from hitting against each other, that allows the holes of the containers to be closed and that provides a certain level of tolerance with regard to the incline of the containers.

For this purpose, the invention proposes a handling assembly capable of handling a container having a hole opening out via an aperture having a perimeter, the handling assembly comprising a suction cup and a negative pressure transfer means; the suction cup having:
- a first surface portion for coming into at least partial contact with the negative pressure transfer means,
- a second surface portion having a contact portion intended to be in at least partial contact with the perimeter of the aperture of the hole of the container,
- first assembly means intended for assembling the suction cup with the negative pressure transfer means,
- through channels between the first surface portion of the suction cup and the second surface portion of the suction cup, and each having:
   * a first aperture opening out onto the first surface portion of the suction cup, and intended to be in fluid communication with a duct of the negative pressure transfer means,
   * a second aperture opening out onto the contact portion, and
- a protrusion on the second surface portion of the suction cup,
   * protruding in relation to the contact portion,
   * surrounded at least partially by the contact portion, and
   * intended to close the hole of the container;
   the negative pressure transfer means having:
- a first surface portion for coming into at least partial contact with a vacuum conditioning system,
- a second surface portion for coming into at least partial contact with the suction cup,
- second assembly means intended for assembling the negative pressure transfer means with the suction cup, and
- a duct having:
   * a first aperture opening out onto the first surface portion so as to be in fluid communication with the vacuum conditioning system, and
   * a second aperture opening out onto the second surface portion so as to be in fluid communication with channels of the suction cup;
the suction cup and the negative pressure transfer means being intended to be assembled in a hermetically-sealed manner by the first assembly means of the suction cup and the second assembly means of the negative pressure transfer means, such that the first apertures of the channels of the suction cup are in fluid communication with the second aperture of the duct of the negative pressure transfer means.

The suction cup according to the invention is designed such that the channels are in fluid communication with a vacuum conditioning system. By positioning the two apertures of the channels on the perimeter of the aperture of the hole of the container such that the protrusion closes the hole of the container, and by producing a vacuum in the channels, the atmospheric pressure holds the perimeter of the aperture of the hole on the contact portion of the suction cup. This ensures the leakproof sealing of the join between the perimeter of the aperture of the hole and the contact portion and guarantees the closing of the hole of the container by the protrusion. The container can thus be handled via the suction cup.

If multiple containers are handled by a system comprising multiple suction cups, given that each suction cup corresponds to one container, containers initially present in a box remain in their respective positions with regard to each other and do not run the risk of hitting against each other.

Moreover, the suction cup can be inclined, at least to a slight degree, in any direction whatsoever, without the container being released.

Given that the protrusion closes the hole of the container, it allows a vacuum applied by the channels of the suction cup to not be applied to a liquid contained in the hole of the container.

The suction cup enables a single container to be taken from the centre of a box comprising numerous containers, or enables containers of a box to be taken row by row as each suction cup corresponds to a single container.

The suction cup according to the invention can, for example, be a crown or ring suction cup.

Within the scope of this document, a "container" can be, for example, a syringe, a beaker, a flask, a bottle or a test tube. The hole of the container preferably only has a single aperture, which can be blocked by the protrusion of the suction cup.

Within the scope of this document, a "perimeter" of an aperture of a hole of a container can include a collar, an edge of the aperture of the hole or a neck. The hole of the container can also be referred to as the "cavity of the container".

Within the scope of this document, a "fluid communication" between elements can be direct or indirect, i.e. take place via other elements. It is preferably sealed, i.e. without the intake or output of fluid between the elements in fluid communication, although small leaks can be tolerated. Fluid communication allows a gas to be transmitted, and thus a gas pressure to be transmitted, and therefore allows a vacuum to be transmitted by suctioning the gas.

Within the scope of this document, a "vacuum conditioning system" is any system, device or set of elements allowing for the creation of the vacuum, i.e. allowing for the suctioning of the gas. It can, for example, include a tray, as described herein. It can also include a vacuum pump.

The first surface portion of the suction cup preferably comprises a first end of the suction cup. The second surface portion of the suction cup preferably comprises a second end of the suction cup opposite the first end.

The negative pressure transfer means according to the invention enables the junction to be made between the suction cup and the vacuum conditioning system. As the suction cup is preferably made from a flexible material, the negative pressure transfer means, which is preferably made from a more rigid material than that of the suction cup, allows for the easy securing of the suction cup to the vacuum conditioning system, in particular to a tray forming part of the vacuum conditioning system.

Moreover, in order to secure numerous suction cups to the vacuum conditioning system, each suction cup can be easily secured to its negative pressure transfer means, for example by interlocking if the suction cup is made from a flexible material, then the pressure transfer means can be easily secured, for example by screwing, to the vacuum conditioning system.

The negative pressure transfer means can, where necessary, be referred to as the "pressure transfer means", the "suction transfer means", the "gas transfer means", the "fluid connection means" or the "vacuum transfer means".

The first surface portion of the negative pressure transfer means preferably comprises a first end of the negative pressure transfer means. The second surface portion of the negative pressure transfer means preferably comprises a second end of the negative pressure transfer means, opposite the first end.

Preferably, in the handling assembly, the first surface portion of the suction cup is in contact with the second surface portion of the negative pressure transfer means.

The hermetically-sealed assembly between the negative pressure transfer means and the suction cup allows the negative pressure created by the vacuum conditioning system to be propagated, through the duct of the negative pressure transfer means and the channels of the suction cup, as far as the contact portion of the second surface portion of the suction cup, where it enables the perimeter of the container to be suctioned.

The suction cup according to the invention is preferably made from a flexible material. For example, the suction cup can be made from a material having a Young Modulus between 1 and 1000 MPA, preferably between 1 and 100 MPA. This flexibility allows the suction cup to adapt to suit the shape of the negative pressure transfer means, so that the connection between the container and the negative pressure transfer means is hermetically sealed. This flexibility further allows the suction cup to be deformed to receive a portion of the negative pressure transfer means.

Advantageously, the negative pressure transfer means is made from a material that is more rigid than that of the suction cup.

Preferably, the suction cup is made from a material at least partially comprising silicone. More preferably, the suction cup is made entirely from silicone.

Silicone has the appropriate rigidity to allow for a slight deformation of the suction cup when the vacuum is applied in order for the suction cup to be optimally pressed against the perimeter of the aperture of the hole. Moreover, it can be sterile.

In one embodiment of the invention, the suction cup is made in one piece.

This simplifies its manufacture. This also makes it particularly solid.

In one embodiment of the invention, the negative pressure transfer means is made in one piece.

This simplifies its manufacture. This also makes it particularly solid.

Advantageously, the contact portion is included in a recess
- intended to receive at least one portion of the perimeter of the aperture of the hole of the container, and
- into which the second apertures of the channels open out.

The recess allows for the easy positioning of the perimeter of the aperture of the hole in relation to the contact portion, and therefore of the hole in relation to the protrusion. Furthermore, it results in a good grip between the perimeter of the aperture of the hole of the container and the contact portion of the suction cup.

In one embodiment of the invention, the first assembly means comprise a hollow of the suction cup intended to place the first apertures of the channels in fluid communication with the duct of the negative pressure transfer means, the hollow having:
-- a narrow portion intended to receive a narrow portion of the negative pressure transfer means, and
-- a wide portion
   ** intended to receive a wide portion of the negative pressure transfer means,
   ** closer to the channels than the narrow portion of the hollow,
   ** having a cross-sectional area that is larger than a cross-sectional area of the narrow portion of the hollow so as to be able to block the wide portion of the negative pressure transfer means.

Such assembly means allow for an easy, fast, reliable assembly that can easily be undone. Within the scope of this document, a "cross-section" of an element, for example of a hollow, is a cross-section along a horizontal plane when the suction cup and the negative pressure transfer means are assembled vertically on top of each other.

In one embodiment of the invention, the second assembly means comprise:
- a narrow portion intended to be received in a narrow portion of a hollow of the suction cup, and
- a wide portion
   * intended to be received in a wide portion of the hollow of the suction cup,
   * closer to the second aperture of the duct than the narrow portion of the negative pressure transfer means, and
   * having a cross-sectional area that is larger than a cross-sectional area of the narrow portion of the negative pressure transfer means.

Such assembly means allow for an easy, fast, reliable assembly with the suction cup that can easily be undone.

Preferably, the negative pressure transfer means further comprises supports intended to bear a protrusion located on a second surface portion of the suction cup, the supports being separated by distribution lines placing the second aperture of the duct in fluid communication with a peripheral line intended to be in fluid communication with the channels of the suction cup.

The supports prevent a deformation of the suction cup as a result of a difference in pressure, which could prevent the hole of the container from being closed by the protrusion and/or separate the second channel apertures of the perimeter of the aperture of the hole, and thus result in the release of the container.

Preferably, the handling assembly further comprises a suction air saving device assembled with the negative pressure transfer means.

If multiple handling assemblies are connected to the same vacuum conditioning system, so as to grasp multiple containers in parallel, a handling assembly may become incorrectly positioned in relation to the perimeter of the aperture of the hole of a container, or a container may be missing. The vacuum conditioning device would thus aspirate ambient air via the channels of the suction cup of the corresponding handling assembly, which would result in a loss of negative pressure. The suction air saving device reduces the fluid passage from these channels opening into the ambient air. It thus reduces the loss of negative pressure in such a situation.

According to a second aspect, the invention proposes a tray for bearing the handling assemblies and transmitting to them a pressure, and comprising:
- a first surface portion,
- a second surface portion having a contact portion for coming into at least partial contact with the handling assemblies,
- a vacuum chamber located between the first and second surface portions,
- an inlet opening out onto the first surface portion and into the vacuum chamber and placing the vacuum chamber in fluid communication with a vacuum pump,
- channels, each opening out on the one hand into the vacuum chamber and on the other hand onto the contact portion so as to be in fluid communication with the handling assemblies, and
- means for securing the handling assemblies onto the contact portion.

The tray allows for the handling, in one action and in parallel, of numerous containers via handling assemblies.

Preferably, the tray further comprises a pressure redistribution wall located in the vacuum chamber opposite the inlet.

The pressure redistribution wall allows for the distribution of the suction originating from the vacuum conditioning system between the channels of the tray, in order to prevent channels of the tray located directly opposite the inlet of the tray from experiencing higher suction than the off-centre trays.

In a preferred manner, the tray comprises a polymeric resin.

This allows the tray to be manufactured via a stereolithographic method, enabling the creation of a relatively thin tray with a fine structure, in particular for the vacuum chamber and the channels of the tray.

In one embodiment of the invention, the means for securing the handling assemblies comprise inserts, preferably metallic, incorporated into the polymeric resin.

These inserts make the attachment to the handling assemblies highly reliable, for example if each of the latter comprises a threaded duct.

In one embodiment of the invention, the tray comprises at least one fin intended to be received in a notch of a nest for syringes.

A nest usually has at least one notch to allow for easy manual handling. In particular, a nest can have two offset notches. The one or more fins on the tray provide for the direct alignment of the tray on the nest and thus of the handling assemblies secured to the tray on the collars of the syringes contained in the nest.

According to a third aspect, the invention proposes a container grasping system comprising:
- the tray according to the invention, and
- handling assemblies according to the invention, secured to the tray such that the second apertures of the channels of the suction cup can be placed in fluid communication with a vacuum pump via the channels of the suction cup, the duct of the negative pressure transfer means, and the tray.

The container grasping system preferably only comprises a single machine. This reduces the volume and cost in relation to known container grasping systems that require more than one machine.

Moreover, it provides for a fast nest unloading rate as all syringes can be grasped in parallel.

The tray can potentially include a means for closing certain channels of the tray in order to choose which containers are taken by the container grasping system.

It is interesting to note that the use of this invention is not limited to the unloading of containers from a nest. Indeed, the invention can also be used for any handling of containers, for example, to load a machine to manufacture blisters or any machine handling syringes, flasks, bottles or test tubes.

### Brief description of the figures

Other characteristics and advantages of the invention shall be better understood upon reading the following detailed description given with reference to the appended figures, in which:
- figure 1 is a three-dimensional view substantially from below of a suction cup in one embodiment of the invention,
- figure 2 is a three-dimensional view substantially from above of a suction cup in one embodiment of the invention,
- figure 3 is a cross-section along a vertical plane of a suction cup in one embodiment of the invention,
- figure 4 is a three-dimensional view substantially from below of a negative pressure transfer means in one embodiment of the invention,
- figure 5 is a cross-section along a vertical plane of a negative pressure transfer means in one embodiment of the invention,
- figure 6 is a side view of a negative pressure transfer means in one embodiment of the invention,
- figure 7 is a cross-sectional view showing a handling assembly in one embodiment of this invention,
- figure 8 is a cross-section along a vertical plane showing a suction cup in one embodiment of the invention, positioned on a container,
- figure 9 is a cross-section along a vertical plane showing a suction cup in one embodiment of the invention, positioned on another container,
- figure 10 shows a cross-section along a vertical plane of a tray for bearing handling assemblies in one embodiment of the invention,
- figure 11 illustrates a container grasping system in one embodiment of the invention, and
- figure 12 shows a cross-section along a vertical plane of a handling assembly comprising a suction cup in another embodiment of the invention.

### Embodiments of the invention

This invention is described with specific embodiments and references to figures, however the invention is not limited thereto. The drawings or figures described are only schematic and are not limiting.

Within the context of this document, the terms "first" and "second" are only used to differentiate between the different elements and do not indicate an order established between said elements.

In the figures, identical or similar elements can keep the same reference numbers.

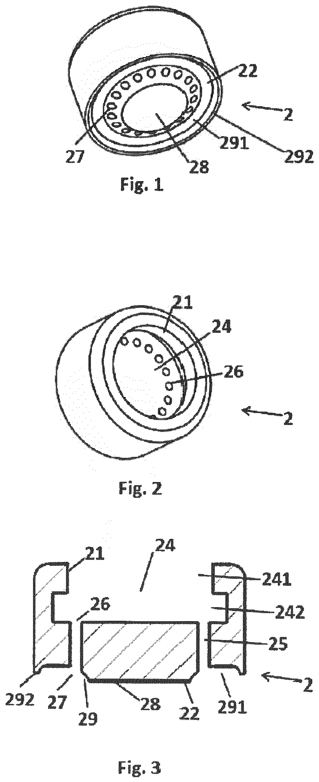

Figures 1, 2 and 3 illustrate a suction cup 2 in one embodiment of the invention. Figure 1 is a three-dimensional view substantially from below. Figure 2 is a three-dimensional view substantially from above. Figure 3 is a cross-section along a vertical plane.

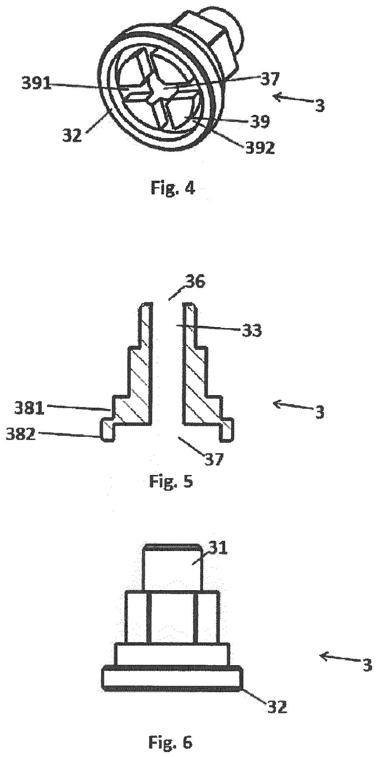

The suction cup 2 is intended to be assembled with a negative pressure transfer means such as a negative pressure transfer means 3 according to the invention, for example that illustrated in figures 4 to 6. This assembly can be produced by first assembly means provided on the suction cup 2. The suction cup 2 is intended to handle, for example to grasp, a container having a hole opening out via an aperture having a perimeter situated around an aperture of the hole.

The suction cup 2 comprises a first surface portion 21 for coming into at least partial contact with the negative pressure transfer means 3, and a second surface portion 22 having a contact portion 29 intended to be in at least partial contact with the perimeter of the aperture of the hole of the container.

In one embodiment of the invention, the first surface portion 21 of the suction cup 2 can substantially comprise an inner surface of the suction cup 2, for example the surface defining a hollow 24. In one embodiment of the invention, the first surface portion 21 of the suction cup 2 can be substantially oriented towards the negative pressure transfer means 3 when the latter is assembled with the suction cup 2.

In one embodiment of the invention, the second surface portion 22 of the suction cup 2 can substantially comprise an outer surface of the suction cup 2. In one embodiment of the invention, the second surface portion 22 of the suction cup 2 can be substantially oriented towards the container when the latter is grasped by the suction cup 2.

The suction cup 2 further comprises through channels 25 between the first surface portion 21 and the second surface portion 22. Each channel 25 has a first aperture 26 opening out onto the first surface portion 21 of the suction cup 2 and a second aperture 27 opening out onto the contact portion 29 of the suction cup 2. The first aperture 26 is intended to be in fluid communication with a duct 33 of the negative pressure transfer means 3, and the second aperture 27 is intended to be joined to the perimeter of the aperture of the hole of the container so that a vacuum present in the duct results in the perimeter being pressed against all of the second apertures 27 of the ducts.

The suction cup 2 further comprises a protrusion 28 on the second surface portion 22, which protrudes in relation to the contact portion 29 and is surrounded at least partially by the contact portion 29. Thus, a perimeter of the protrusion 28 is in contact with a perimeter of the aperture of the hole of the container when the second apertures 27 of the channels 25 are joined to the perimeter of the aperture of the hole of the container, such that the protrusion 28 closes the hole of the container. The protrusion 28 is preferably central on the second surface portion 22.

In a preferred manner, the contact portion 29 is included in a recess 291, into which open out the second apertures 27 of the channels 25 and is intended to receive at least one portion of the perimeter of the aperture of the hole of the container. The recess 291 can, for example, be created by the protrusion 28 and an edge 292. The recess 291 improves the blockage of the perimeter of the aperture of the hole on the suction cup 2 by minimising lateral movements of the perimeter of the aperture of the hole in relation to the suction cup 2.

Preferably, the suction cup 2 comprises a hollow 24 in which first apertures 26 of the channels 25 are located and in which a portion of the negative pressure transfer means 3 is blocked, such that the duct 33 of the negative pressure transfer means 3 is in fluid communication with the channels 25.

For example, in one embodiment of the invention, the hollow 24 forms a part of the first assembly means. The hollow 24 can, for example, include a narrow portion 241 intended to receive a narrow portion 381 (figure 5) of the negative pressure transfer means 3, and a wide portion 242 intended to receive a wide portion 382 (figure 5) of the negative pressure transfer means 3, the wide portion 242 of the hollow being closer to the channels 25 than the narrow portion 241 of the hollow 24.

Given that the wide portion 242 of the hollow has a cross-sectional area that is larger than a cross-sectional area of the narrow portion 241 of the hollow 24, the wide portion 382 (figure 5) of the negative pressure transfer means 3 can thus be blocked in the hollow 24 via interlocking.

The dimensions and the shape of the suction cup 2 are preferably chosen to suit the shape and the dimensions of the container to be handled. For example, in order to handle a container having a circular hole, the protrusion 28 is preferably circular. Moreover, by way of example, in order to handle a syringe having a diameter of 8.15 mm, the suction cup 2 can have a cylindrical outer surface that is 14.5 mm in diameter and the protrusion 28 can be circular with a diameter of 6.85 mm.

It is interesting to note that the perimeter of the aperture of the hole does not need to be flat for the suction cup to grasp and handle the container.

The suction cup 2 can comprise between 2 and 100 channels 25, preferably between 4 and 50 channels, more preferably between 6 and 25 channels.

Figures 4, 5 and 6 illustrate a negative pressure transfer means 3 in one embodiment of the invention. Figure 4 is a three-dimensional view substantially from below. Figure 5 is a cross-section along a vertical plane. Figure 6 is a side view.

The negative pressure transfer means 3 is intended to be assembled with a suction cup such as a suction cup 2 according to the invention, for example that illustrated in figures 1 to 3. This assembly can be produced by second assembly means provided on the negative pressure transfer means 3.

The negative pressure transfer means 3 comprises a first surface portion 31 for coming into at least partial contact with a vacuum conditioning system and a second surface portion 32 for coming into at least partial contact with a suction cup 2. In one embodiment of the invention, the first surface portion 31 of the negative pressure transfer means 3 can substantially comprise an outer surface of the negative pressure transfer means 3. In one embodiment of the invention, the second surface portion 32 of the negative pressure transfer means 3 can substantially comprise an outer surface of the negative pressure transfer means 3.

The negative pressure transfer means 3 comprises a duct 33 having a first aperture 36 opening out onto the first surface portion 31 of the negative pressure transfer means 3 and a second aperture 37 opening out onto the second surface portion 32 of the negative pressure transfer means 3. The duct 33 can thus be in fluid communication with a vacuum conditioning system via its first aperture 36 and with channels 25 of the suction cup 2 via its second aperture 37.

The negative pressure transfer means 3 preferably comprises supports 39 intended to be in contact with the rear of the protrusion 28 of the suction cup 2. The supports 39 enable the protrusion 28 to be supported when an assembly comprising the negative pressure transfer means 3 and the suction cup 2 is in contact, via the suction cup 2, with the container, and when a vacuum is applied in the channels 25 of the suction cup 2 via the duct 33 of the negative pressure transfer means 3. Indeed, without the supports 39, the portion of the suction cup 2 that is internal in relation to the channels 25 can tend to rise, preventing the protrusion 28 from closing the hole and placing the channels 25 in fluid contact with the hole.

The supports 39 are preferably distributed about the second aperture 37 of the duct 33. The supports 39 are separated from each other by distribution lines 391, which are for example radial. In a preferred manner, the distribution lines 391 open out on the one hand onto the second aperture 37 of the duct 33 and on the other hand onto a peripheral line 392 intended to be in fluid communication with the channels 25 of the suction cup 2.

In one embodiment of the invention, the supports 39 are bosses on the second surface portion 32 of the negative pressure transfer means 3, and the distribution lines 391 and the peripheral line 392 are grooves on the second surface portion 32 of the negative pressure transfer means 3.

The shape of the negative pressure transfer means 3 can be chosen so as to supply the second assembly means. For example, in one embodiment of the invention, the negative pressure transfer means 3 has a narrow portion 381 intended to be received in a narrow portion 241 (figure 3) of a hollow 24 of the suction cup 2 and a wide portion 382 intended to be received in a wide portion 242 (figure 3) of the hollow 24 of the suction cup 2, the wide portion 382 of the negative pressure transfer means 3 being closer to the second aperture 37 of the duct 33 than the narrow portion 381 of the negative pressure transfer means 3. The wide portion 382 of the negative pressure transfer means 3 has a cross-sectional area that is larger than a cross-sectional area of the narrow portion 381 of the negative pressure transfer means 3, so as to be able to be blocked in the wide portion 242 of the hollow 24 of the suction cup 2.

The negative pressure transfer means 3 can comprise, on or near to its first surface portion 31, means for fastening to the vacuum conditioning system, for example a screwing thread and a drive, for example a hexagonal drive, for a screwing tool.

The dimensions and the shape of the negative pressure transfer means 3 are preferably chosen to suit the dimensions and the shape of the suction cup 2, which are themselves designed to suit the dimensions of the container to be handled. For example, in order to handle a suction cup 2 having a cylindrical outer surface with a diameter of 14.5 mm, one circular end of the negative pressure transfer means 3 comprising the second surface portion 32 can have a diameter of 12 mm. In such a case, the wide portion 382 of the negative pressure transfer means 3 can be circular and have a diameter of 12 mm, and the narrow portion 381 of the negative pressure transfer means 3 can be circular and have a diameter of 9.8 mm.

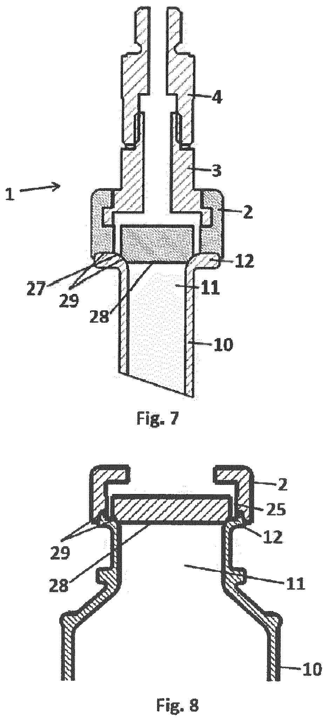

Figure 7 is a cross-sectional view showing a handling assembly 1 in one embodiment of the invention. The handling assembly 1 preferably comprises the suction cup 2 according to the invention and the negative pressure transfer means 3 according to the invention, assembled in a hermetically-sealed manner by the first assembly means of the suction cup 2 and the second assembly means of the negative pressure transfer means 3.

Preferably, the handling assembly 1 comprises a suction air saving device 4 assembled with the negative pressure transfer means 3, preferably in a hermetically-sealed manner. The suction air saving device 4 is positioned so as to reduce a fluid passage from the negative pressure transfer means 3 in the event that the channels 25 of the suction cup 2 are open to the ambient air. In one embodiment of the invention, the suction air saving device 4 is a vacuum saving valve, for example from the ZP2V series marketed by the company SMC.

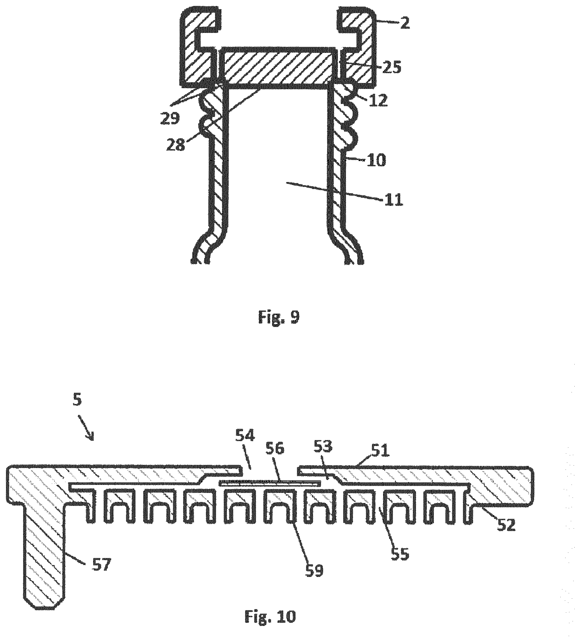

The suction air saving device 4 preferably comprises means for assembly with the negative pressure transfer means 3 and means for assembly with a tray capable of bearing several handling assemblies, for example a tray 5 according to the invention such as the tray 5 illustrated in figure 10. A gasket can be used for assembling the suction air saving device with the negative pressure transfer means 3. A gasket can be used for assembling the suction air saving device with the tray.

In one embodiment of the invention, the negative pressure transfer means 3 is directly secured to such a tray 5, without passing via a suction air saving device 4.

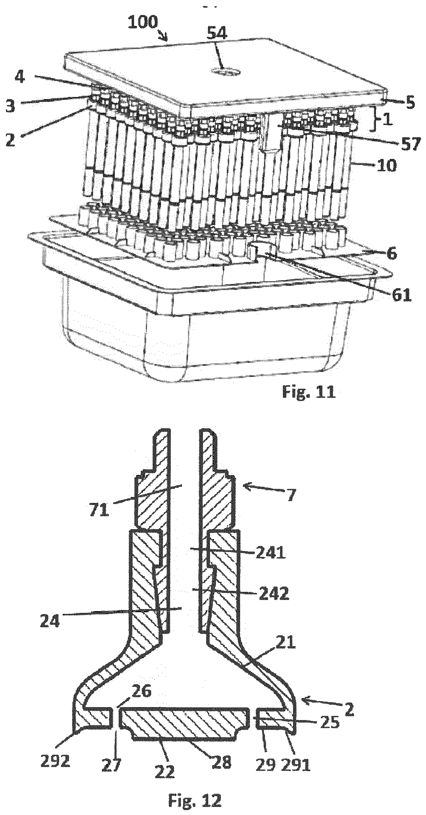

In one embodiment of the invention, the handling assembly 1 comprises the suction cup 2 according to the invention and another negative pressure transfer means (figure 12), assembled in a hermetically-sealed manner, and potentially a suction air saving device 4.

Figure 7 also illustrates a portion of the container 10 handled via the suction cup 2. The container 10 has a hole 11 and a perimeter 12 around an aperture of the hole 11. When the container 10 is handled via the suction cup 2, the contact portion 29 of the suction cup 2 is in contact with the perimeter 12 of the aperture of the hole 11 of the container 10, the second apertures 27 of the channels 25 are joined to the perimeter 12 of the aperture of the hole 11 of the container 10, and the protrusion 28 plugs the hole 11 of the container 10. The container 10 can contain a liquid. Figure 7 illustrates a case in which the perimeter 12 of the aperture of the hole 11 of the container 10 comprises a collar, for example if the container is a syringe.

The perimeter 12 of the aperture of the hole 11 of the container 10 is preferably oblong in shape. The perimeter 12 of the aperture of the hole 11 of the container 10 can be circular, oval or polygonal in shape, etc.

The suction cup 2 is preferably made from a material at least partially comprising silicone, and more preferably is essentially made from silicone. Silicone has the appropriate rigidity to allow for a slight deformation of the suction cup 2 when the vacuum is applied in order for the suction cup 2 to be optimally pressed against the perimeter 12 of the aperture of the hole 11 of the container 10. Moreover, this rigidity allows the wide portion 382 of the negative pressure transfer means 3 (figure 5) to be inserted into the wide portion 242 of the suction cup 2 (figure 3) via the narrow portion 241 of the suction cup 2 (figure 3).

The negative pressure transfer means 3 can comprise aluminium, potentially anodised, or be made from aluminium, potentially anodised. The negative pressure transfer means 3 can comprise stainless steel or be made from stainless steel, which is particularly advantageous if it must be sterile. The negative pressure transfer means 3 is preferably made in one piece.

Figures 8 and 9 are cross-sections along a vertical plane showing a suction cup 2 in one embodiment of the invention, positioned on a container 10. Figure 8 illustrates a case in which the perimeter 12 of the aperture of the hole 11 of the container 10 comprises a slightly flared neck, for example if the container is a flask. Figure 9 illustrates a case in which the perimeter 12 of the aperture of the hole 11 of the container 10 does not comprise a flared portion, for example if the container is a test tube.

Figure 10 shows a cross-section along a vertical plane of a tray 5 for bearing handling assemblies 1 in one embodiment of the invention. The tray 5 is preferably designed such that handling assemblies can be secured thereto, for example handling assemblies 1 according to this invention, such as that illustrated in figure 7. The tray 5 can be designed such that handling assemblies can be secured thereto, such as that illustrated in figure 12.

The tray 5 comprises a first surface portion 51 and a second surface portion 52 having a contact portion 59 for coming into at least partial contact with the handling assemblies 1. The contact portion 59 of the tray 5 can comprise separate surface portions. The tray 5 further comprises a vacuum chamber 53 located between the first 51 and the second 52 surface portions and an inlet 54 opening out onto the first surface portion 51 and into the vacuum chamber 53. The inlet 54 places the vacuum chamber 53 in fluid communication with a device outside of the tray used to aspirate, for example a vacuum pump.

The tray 5 further comprises channels 55 opening out on the one hand into the vacuum chamber 53 and on the other hand onto the contact portion 59 so as to place the vacuum chamber 53 in fluid communication with handling assemblies 1 secured to the contact portion 59.

Preferably, the tray 5 comprises a pressure redistribution wall 56 located in the vacuum chamber 53 opposite the inlet 54.

In one embodiment of the invention, the tray 5 is manufactured by stereolithography and comprises a material that is suited to this manufacturing method, such as a polymeric resin.

The handling assemblies are secured via fastening means provided on the contact portion 59 of the tray 5. The fastening means can, for example, include inserts, preferably metallic, integrated into the polymeric resin during manufacture of the tray. These inserts can comprise threads corresponding to the threads present on the handling assemblies.

Furthermore, the tray 5 can comprise at least one fin 57, preferably on its second surface portion 52 intended to be received in a notch 61 of a nest 6 for syringes (figure 11).

When the suction cup 2 is assembled with the negative pressure transfer means 3, itself secured to the tray 5 connected to the vacuum conditioning system, the creation of a vacuum in the vacuum conditioning system results in the pumping of air into the chamber 54 of the tray 5, in the channels 55 of the tray 5, in the duct 33 of the negative pressure transfer means 3 and in the channels 25 of the suction cup 2. By positioning the container 10 such that the aperture of its hole 11 is blocked by the protrusion 28, the perimeter 12 of the aperture of the hole 11 of the container 10 is bonded to the contact portion 29 of the suction cup 2 by atmospheric pressure.

In order to release the container 10 from the suction cup 2, the vacuum can be shut off or a certain quantity of air can be injected into the chamber of the tray 54 that communicates with the channels 25 and releases the container 10.

Figure 11 is a three-dimensional view of a container grasping system 100 in one embodiment of the invention, comprising the tray 5 and handling assemblies 1 secured to the latter. Figure 11 further shows a nest 6 in which syringes, which are containers 10, are initially inserted, before being grasped by the container grasping system 100. The nest 6 can have one or more notches 61 into which can be inserted the one or more fins 57 of the tray 5.

The tray 5 can further comprise temporary fastening means, for example suction cups, for grasping the nest 6, for example one in each of the corners thereof.

In one embodiment of the invention, the nest 6 comprises 160 syringes, and 160 handling assemblies 1 can be secured onto the tray 5.

In one embodiment of the invention, the container grasping system 100 is sterile.

Figure 12 shows a cross-section along a vertical plane of a handling assembly comprising a suction cup 2 in another embodiment of the invention. In this embodiment of the invention, the suction cup 2 is intended to be assembled with a standard fastening device 7 such as those commercially available, and that has a pressure transfer function via a duct 71. This assembly can be produced by first assembly means provided on the suction cup 2. The assembly comprising the suction cup 2 and the standard fastening device 7 is a handling assembly that can be secured to a tray 5 according to the invention.

In one embodiment of the invention, the first assembly means comprise the hollow 24 having a narrow portion 241 intended to receive a narrow portion of the standard fastening device 7, and a wide portion 242 intended to receive a wide portion of the standard fastening device 7, the wide portion 242 of the hollow being closer to the channels 25 than the narrow portion 241 of the hollow 24.

In other words, the invention relates to a suction cup 2, a negative pressure transfer means 3, a handling assembly 1 comprising the suction cup 2 and the negative pressure transfer means 3, a tray for securing the handling assemblies 1 and a container grasping system 10 comprising the tray and handling assemblies 1.

The suction cup 2 comprises channels that are secured by suction onto a perimeter 12 of an aperture of a hole 11 of the container 10 and a protrusion 28 positioned internally in relation to the channels 25 that closes a hole 11 of the container 10. The suction cup 2 enables the container 10 to be grasped and handled while closing its hole 11. The use of numerous suction cups 2 in parallel enables numerous containers 10 to be handled in parallel.

Independently from any other element, this invention in particular concerns a suction cup 2 for a handling assembly 1 capable of handling a container 10 having a hole 11 opening out via an aperture having a perimeter 12, the suction cup 2 having:
- a first surface portion 21 for coming into at least partial contact with a negative pressure transfer means 3,
- a second surface portion 22 having a contact portion 29 intended to be in at least partial contact with the perimeter 12 of the aperture of the hole 11 of the container 10,
- first assembly means intended for assembling the suction cup 2 with the negative pressure transfer means 3,
- through channels 25 between the first surface portion 21 of the suction cup 2 and the second surface portion 22 of the suction cup 2, and each having:
   -- a first aperture 26 opening out onto the first surface portion 21 of the suction cup 2, and intended to be in fluid communication with a duct 33 of the negative pressure transfer means 3,
   -- a second aperture 27 opening out onto the contact portion 29, and
- a protrusion 28 on the second surface portion 22 of the suction cup 2,
   -- protruding in relation to the contact portion 29,
   -- surrounded at least partially by the contact portion 29, and
   -- intended to close the hole 11 of the container 10.

Said suction cup can have at least one of the characteristics described herein.

Independently from any other element, this invention in particular concerns a negative pressure transfer means 3 for a handling assembly 1 capable of handling a container 10 having a hole 11 opening out via an aperture having a perimeter 12, the negative pressure transfer means 3 having:
- a first surface portion 31 for coming into at least partial contact with a vacuum conditioning system,
- a second surface portion 32 for coming into at least partial contact with a suction cup 2,
- second assembly means intended for assembling the negative pressure transfer means 3 with the suction cup 2, and
- a duct 33 having:
   -- a first aperture 36 opening out onto the first surface portion 31 so as to be in fluid communication with the vacuum conditioning system, and
   -- a second aperture 37 opening out onto the second surface portion 32 so as to be in fluid communication with channels 25 of the suction cup 2.

Said negative pressure transfer means can have at least one of the characteristics described herein.

This invention has been described in relation to specific embodiments, which are provided for illustration purposes only and must not be considered to be limiting. In a general manner, this invention is not limited to the examples illustrated and/or described above. The use of the verbs "contain", "include", "comprise", or any other alternative, as well as their conjugations, can in no way rule out the presence of elements other than those stipulated. The use of the indefinite article "a" or "an", or of the definite article "the" to introduce an element does not rule out the presence of a plurality of said elements. The reference numbers in the claims do not limit the scope thereof.

## Claims

1. Handling assembly (1) capable of handling a container (10) having a hole (11) opening out via an aperture having a perimeter (12), the handling assembly (1) comprising a suction cup (2) and a negative pressure transfer means (3); the suction cup (2) having:
- a first surface portion (21) for coming into at least partial contact with the negative pressure transfer means (3),
- a second surface portion (22) having a contact portion (29) intended to be in at least partial contact with the perimeter (12) of the aperture of the hole (11) of the container (10),
- first assembly means intended for assembling the suction cup (2) with the negative pressure transfer means (3),
- through channels (25) between the first surface portion (21) of the suction cup (2) and the second surface portion (22) of the suction cup (2), and each having:
-- a first aperture (26) opening out onto the first surface portion (21) of the suction cup (2), and intended to be in fluid communication with a duct (33) of the negative pressure transfer means (3),
-- a second aperture (27) opening out onto the contact portion (29), and
- a protrusion (28) on the second surface portion (22) of the suction cup (2),
-- protruding in relation to the contact portion (29),
-- surrounded at least partially by the contact portion (29), and
-- intended to close the hole (11) of the container (10);
the negative pressure transfer means (3) having:
- a first surface portion (31) for coming into at least partial contact with a vacuum conditioning system,
- a second surface portion (32) for coming into at least partial contact with the suction cup (2),
- second assembly means intended for assembling the negative pressure transfer means (3) with the suction cup (2), and
- a duct (33) having:
-- a first aperture (36) opening out onto the first surface portion (31) so as to be in fluid communication with the vacuum conditioning system, and
-- a second aperture (37) opening out onto the second surface portion (32) so as to be in fluid communication with channels (25) of the suction cup (2);
the suction cup (2) and the negative pressure transfer means (3) being intended to be assembled in a hermetically-sealed manner by the first assembly means of the suction cup (2) and the second assembly means of the negative pressure transfer means (3), such that the first apertures (26) of the channels (25) of the suction cup (2) can be in fluid communication with the second aperture (37) of the duct (33) of the negative pressure transfer means (3).

2. Handling assembly (1) according to claim 1, wherein the suction cup (2) is made from a flexible material.

3. Handling assembly (1) according to the previous claim, wherein the negative pressure transfer means (3) is made from a material that is more rigid than that of the suction cup (2).

4. Handling assembly (1) according to any one of claims 2 to 3, wherein the suction cup (2) is made from a material at least partially comprising silicone, preferably made entirely from silicone.

5. Handling assembly (1) according to any one of the previous claims, wherein the suction cup (2) is made in one piece.

6. Handling assembly (1) according to any one of the previous claims, wherein the negative pressure transfer means (3) is made in one piece.

7. Handling assembly (1) according to any one of the previous claims, wherein the contact portion (29) is included in a recess (291)
- intended to receive at least one portion of the perimeter (12) of the aperture of the hole (11) of the container (10), and
- into which the second apertures (27) of the channels (25) open out.

8. Handling assembly (1) according to any one of the previous claims, wherein the first assembly means comprise a hollow (24) of the suction cup (2) intended to place the first apertures (26) of the channels (25) in fluid communication with the duct (33) of the negative pressure transfer means (3), the hollow (24) having:
-- a narrow portion (241) intended to receive a narrow portion (381) of the negative pressure transfer means (3), and
-- a wide portion (242)
--- intended to receive a wide portion (382) of the negative pressure transfer means (3),
--- closer to the channels (25) than the narrow portion (241) of the hollow (24),
--- having a cross-sectional area that is larger than a cross-sectional area of the narrow portion (241) of the hollow (24) so as to be able to block the wide portion (382) of the negative pressure transfer means (3).

9. Handling assembly (1) according to any one of the previous claims, wherein the second assembly means comprise:
- a narrow portion (381) intended to be received in a narrow portion (241) of a hollow (24) of the suction cup (2), and
- a wide portion (382)
-- intended to be received in a wide portion (242) of the hollow (24) of the suction cup (2),
-- closer to the second aperture (37) of the duct (33) than the narrow portion (381) of the negative pressure transfer means (3), and
-- having a cross-sectional area that is larger than a cross-sectional area of the narrow portion (381) of the negative pressure transfer means (3).

10. Handling assembly (1) according to any one of the previous claims, wherein the negative pressure transfer means (3) further comprises supports (39) intended to bear the protrusion (28), the supports (39) being separated by distribution lines (391) placing the second aperture (37) of the duct (33) in fluid communication with a peripheral line (392) intended to be in fluid communication with the channels (25) of the suction cup (2).

11. Handling assembly (1) according to any one of the previous claims, further comprising a suction air saving device (4) assembled with the negative pressure transfer means (3).

12. Tray (5) for bearing handling assemblies (1) according to any one of the previous claims and for transmitting a pressure, and comprising:
- a first surface portion (51),
- a second surface portion (52) having a contact portion (59) for coming into at least partial contact with the handling assemblies (1),
- a vacuum chamber (53) located between the first (51) and second (52) surface portions,
- an inlet (54) opening out onto the first surface portion (51) and into the vacuum chamber (53) and placing the vacuum chamber (53) in fluid communication with a vacuum pump,
- channels (55), each opening out on the one hand into the vacuum chamber (53) and on the other hand onto the contact portion (59) so as to be in fluid communication with the handling assemblies (1), and
- means for securing the handling assemblies (1) onto the contact portion (59).

13. Tray (5) according to the previous claim, further comprising a pressure redistribution wall (56) located in the vacuum chamber (53) opposite the inlet (54).

14. Tray (5) according to any one of claims 12 to 13, comprising a polymeric resin.

15. Tray (5) according to the previous claim, wherein the means for securing the handling assemblies (1) comprise inserts, preferably metallic, incorporated into the polymeric resin.

16. Tray (5) according to any one of claims 12 to 15, further comprising at least one fin (57) intended to be received in a notch (61) of a nest (6) for syringes.

17. Container grasping system (100) comprising:
- a plate (5) according to any one of claims 12 to 16, and
- handling assemblies (1) according to any one of claims 1 to 11, secured to the tray (5) such that the second apertures (27) of the channels (25) of the suction cup (2) can be placed in fluid communication with a vacuum pump via the channels (25) of the suction cup (2), the duct (33) of the negative pressure transfer means (3), and the tray (5).
